# EUROPEAN PATENT APPLICATION

(11) **EP 3 401 682 A1**
(43) Date of publication of application: **14.11.2018**
(21) Application number: 17305525.2
(22) Date of filing: 09.05.2017
(51) Int. Cl.: G01N 33/564, A61K 39/02, A61K 35/741

(54) **DIAGNOSIS, PREVENTION AND TREATMENT OF DEMYELINATING DISORDERS OF THE CENTRAL NERVOUS SYSTEM**

(71) Applicant: Université de Nantes, 44035 Nantes Cedex 1 (FR); INSTITUT NATIONAL DE LA SANTE ET DE LA RECHERCHE MEDICALE (INSERM), 75013 Paris (FR)
(72) Inventor: MONTASSIER, Emmanuel, 44116 Vieillevigne (FR); LE BERRE, Ludmilla, 44100 Nantes (FR); SOULILLOU, Jean-Paul, 44100 Nantes (FR)
(74) Representative: Nony

(57) **Abstract**

The present invention relates to the diagnosis, the prevention and the treatment of demyelinating disorders of the central nervous system (CNS), in particular disorders exhibiting altered levels of antibodies directed against α1-3-galactose, such as multiple sclerosis (MS) and clinically isolated syndrome (CIS). The diagnosis of MS and CIS still suffers from a lack of reliable biomarkers and there is cure for these diseases, except medication acting on the immune component of the overt disease, with the usual side effects of the immunosuppression or anti inflammation intended to relieve the associated symptoms. The inventors showed that there is a significant decrease in the levels of antibodies directed against α1-3-galactose in individuals having a MS or a CIS. In addition, the present invention relates to the prevention and/or the treatment of a demyelinating disorder of the CNS in an individual, comprising a step of administering a compound suitable for generating an immune reaction directed against α1-3-galactose in the said individual. Large cohorts of human individuals having MS or CIS have been studied and compared to matched cohorts on the basis of age and gender.

## Description

### FIELD OF THE INVENTION

The present invention relates to the diagnosis, the prevention and the treatment of a demyelinating disorder of the central nervous system (CNS).

In particular, the invention relies upon the observation that the levels of anti-α1-3 Gal antibodies (referred as anti-Gal thereafter in the text) are decreased in individuals having multiple sclerosis (MS) or clinically isolated syndrome (CIS). Consequently, the level of anti-Gal antibodies may be of use as a biomarker for the diagnosis of demyelinating disorders of the CNS. The corollary of these observations is that therapeutic strategies intended to increase the levels of anti-Gal antibodies in individuals having a demyelinating disorder of the CNS may be considered.

### BACKGROUND OF THE INVENTION

Multiple sclerosis (MS) is an inflammatory demyelinating disease of the central nervous system (CNS) of unknown etiology. Despite the fact that an elusive primary "degenerative" abnormality cannot be ruled out, the disease displays a clear autoimmune component at its overt clinical state reinforced by evidence for genetic linkages to genes affecting antigen presenting cells, including B cells, T cell homing or cytokine receptors. Several viruses have been linked to MS etiology, particularly the Epstein-Barr virus (EBV), which shows a strong association with disease occurrence. The potential role of EBV in initiating the autoimmune disease has been based on the presence of lymphocytes committed to EBV (or to cross reacting antigens) in the CNS, EBV in leptomeningitis lesions and possible effects of EBV infection on blood-brain barrier (BBB) endothelial cells and on the requirement for a past EBV infection for the disease to occur. High titers of anti-EBNA1 antibodies prior to MS onset or at the time of a clinically isolated syndrome as well as a previous history of infectious mononucleosis (IMN) or high anti-EBV titers have also been shown to increase the risk of developing MS. Nevertheless the etiology of the MS disease remains speculative.

The relationship between MS and response to EBV has led to speculation in the context of a "Hygiene theory" of autoimmunity, in which an inappropriate response to infectious agents has developed following a supposed defect in regulation of the immune response which has resulted from new urban environment and particularly the decrease in parasite-related immune regulation.

Particularly relevant is the fact that human beings have a loss-mutation of the gene encoding the α1-3-galactosyltransferase (GGTA1) allowing the synthesis of the α1-3 Gal antigen. Consequently human being, unable to produce the antigen, develop high level of anti α1-3 Gal from the first year of life and in adulthood as the result of an immunisation against the gut's bacteria of their microbiota, which are positive for the GGTA1 gene. Therefore, the identification of new reliable biomarkers for MS and CIS, and particularly those linked to the cross-talk of the patients' immune response and their micro-organism environment would benefit to unravel the etiology of these diseases and would provide a basis for their diagnosis.

In addition, there is no prevention of the disease acting at the etiological level and the current treatments that are administered to date to these patients act on the late autoimmune component of the disease, which is being intended to alleviate the symptoms, such as the inflammation.

Therefore, the identification of new concepts and targets to combat these diseases would benefit the patient's health.

### SUMMARY OF THE INVENTION

A first aspect of the invention relates to a compound suitable for generating an immune reaction directed against α-1,3-galactose in an individual in need thereof for use in treating and/or preventing a demyelinating disorder of the central nervous system (CNS) in said individual.

Another aspect of the invention also relates to a pharmaceutical composition comprising a compound according to the instant disclosure and a pharmaceutical acceptable vehicle.

A further aspect of the invention also relates to a vaccine composition comprising a compound or a pharmaceutical composition according to the instant disclosure and an adjuvant.

In another aspect, the invention further relates to a method for diagnosing a demyelinating disorder of the CNS in an individual comprising the steps of:
- a) measuring the level of antibodies directed against α1-3-galactose in a sample from an individual;
- b) comparing the level of antibodies directed against α1-3-galactose measured at step a) to a reference value of the said level of antibodies directed against α1-3-galactose. Finally, the invention further relates to a method for assessing the efficiency of a treatment of a demyelinating disorder of the CNS in an individual, comprising the steps of:
- a) measuring the level of antibodies directed against α1-3-galactose in a biological sample from an individual prior to a treatment;
- b) measuring the level of antibodies directed against α1-3-galactose in a biological sample from an individual after a treatment;
- c) comparing level value measured at step a) with the level value measured at step b).

### LEGENDS OF THE FIGURES

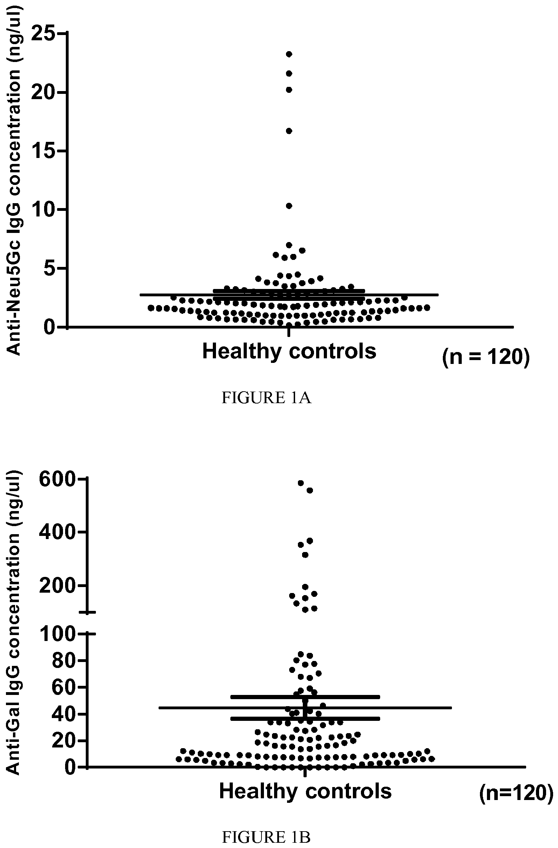

**Figure 1****:** Plots illustrating the anti-Neu5Gc (used as a control specificity in all the Plots described) and anti-Gal IgGs levels in normal individuals. The results are expressed in ng/µl as individual plots with median and interquartile range for anti- Neu5Gc IgG (**A**) and anti-Gal IgG (**B**) (n=120).

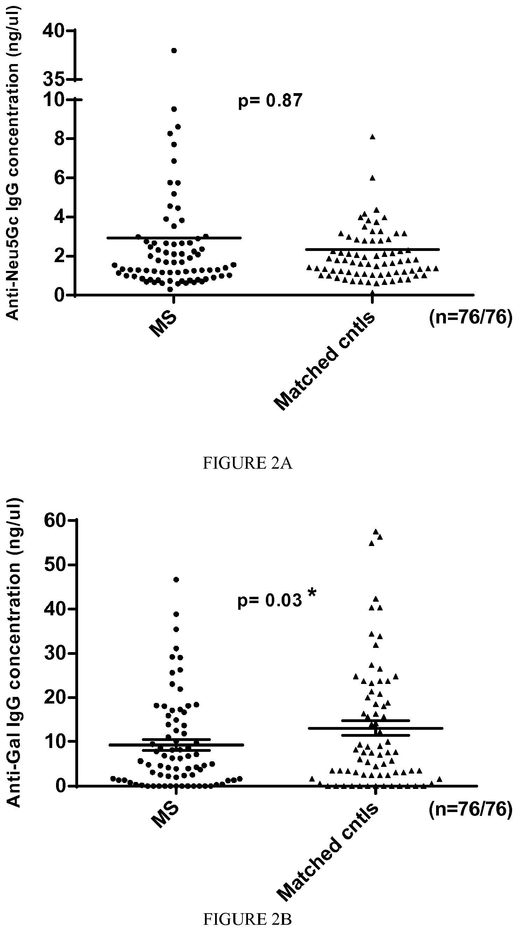

**Figure 2****:** Plots illustrating the anti-Neu5Gc IgGs and anti-Gal IgGs levels in a global MS cohort versus healthy individuals matched on the basis of age and gender criteria (n=76/76). The results are expressed in ng/µl as individual plots with median and interquartile range for each group, for anti-Neu5Gc IgGs (**A**) and anti-Gal IgGs (**B**) (n=76/76). *p<0.05, using a Wilcoxon non-parametric paired test for anti-Neu5Gc IgGs and anti-Gal IgGs.

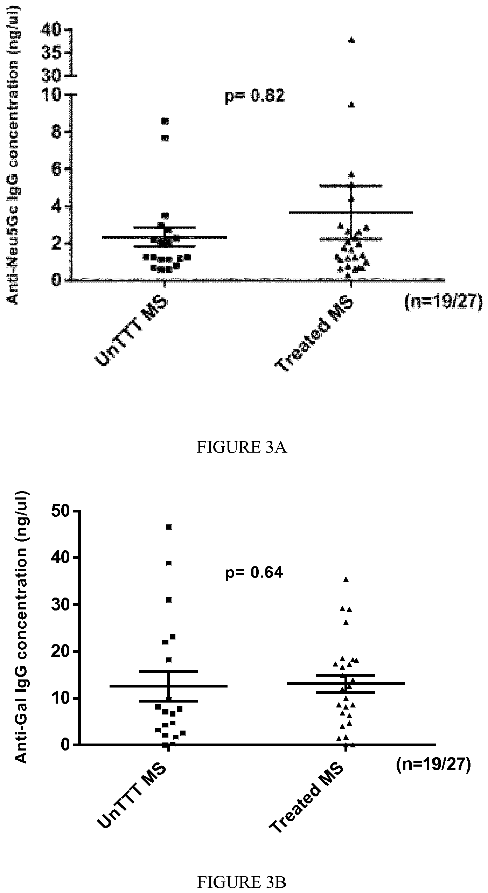

**Figure 3**: Plots illustrating the anti-Neu5Gc IgGs and anti-Gal IgGs in untreated (unTTT MS) versus treated MS patients. The results are expressed in ng/µl as individual plots with median and interquartile range for each group, for anti-Neu5Gc IgGs (**A**) and anti-Gal IgGs (**B**) (n=19/27).

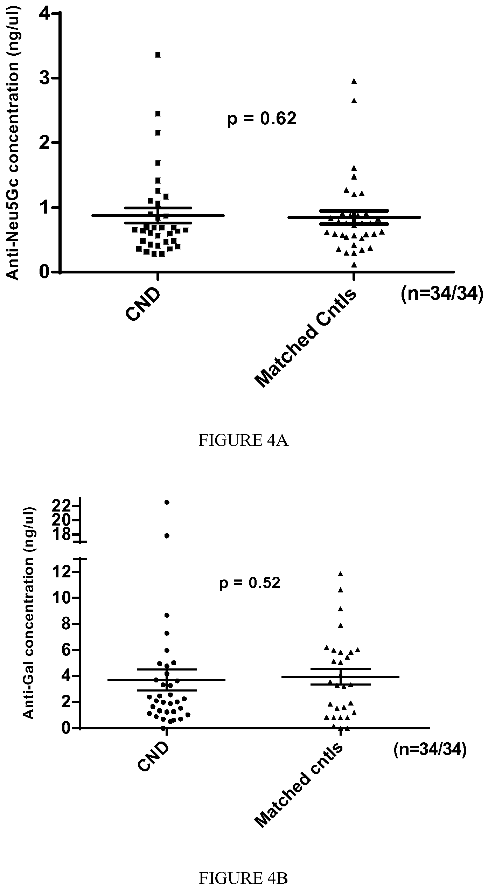

**Figure 4**: Plots illustrating the anti-Neu5Gc IgGs and anti-Gal IgGs levels in patients with non-MS/CIS diseases affecting central nervous system (CNS) versus healthy individuals matched on the basis of age and gender criteria (n=34/34). The results are expressed in ng/µl as individual plots with median and interquartile range for each group, for anti-Neu5Gc IgGs (**A**) and anti-Gal IgGs/Ms (**B**). *p<0.05 using a Wilcoxon non-parametric paired test.

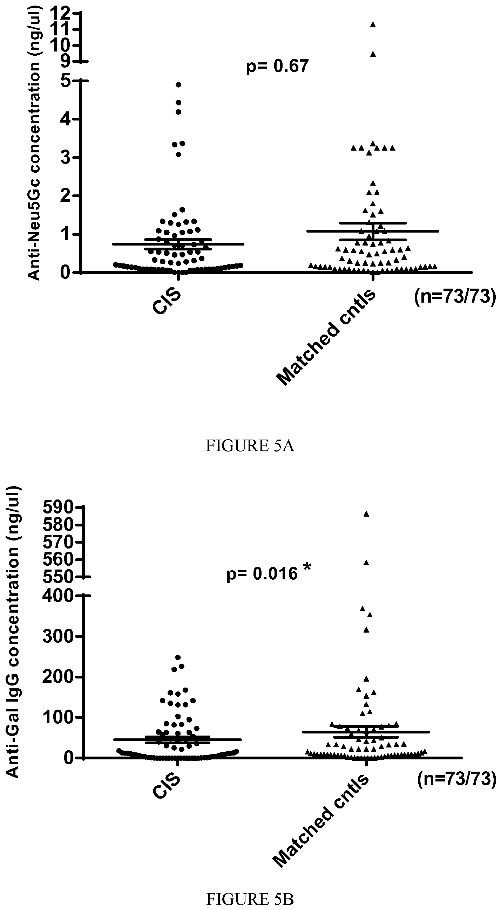

**Figure 5****:** Plots illustrating the anti-Neu5Gc IgGs and anti-Gal IgGs levels in the CIS cohort versus healthy individuals matched on the basis of age and gender criteria (n=73/73). The results are expressed in ng/µl as individual plots with median and interquartile range for each group, for anti-Neu5Gc IgGs (**A**) and anti-Gal IgGs (**B**). *p<0.05, using a Wilcoxon non-parametric paired test for IgGs.

### DETAILED DESCRIPTION OF THE INVENTION

The inventors observed a significant decrease in the levels of circulating anti-Gal antibodies in individuals having MS or CIS. To the inventors' knowledge, said variation was never observed before. Therefore, the level of circulating anti-Gal antibodies may provide a biomarker for the diagnosis of demyelinating disorders of CNS.

Moreover, the inventors provide a rational for taking advantage of compounds bearing α1-3-galactose epitopes or exogenous anti-Gal antibodies in order to restore higher levels of circulating anti-Gal antibodies in order to provide a therapeutic strategy for treating individuals with MS or CIS.

Indeed, anti- α 1,3 Gal have been involved in protection against infectious GGTA1 positive bacteria (e.g. Hamadeh et al. (Clin. Invest. (1992) 89(4):1223-1235)), virus resulting from budding from GGT1A positive cell membrane (Takeuchi et al. (Nature; (1996) 379:85-88)) as well as in some autoimmune phenomena (Galili. Subcell. Biochem. (1999) 32:339-360)), providing a rationale for a role of anti α1-3-Gal antibodies in the cross talk between the human organism and its environment.

### USES

### • Compounds suitable for generating an immune reaction directed against α1-3-galactose

A first aspect of the invention relates to a compound suitable for generating an immune reaction directed against α-1,3-galactose in an individual in need thereof for use in treating and/or preventing a demyelinating disorder of the CNS in said individual.

The instant invention also relates to the use of a compound suitable for generating an immune reaction directed against α1-3-galactose in an individual in need thereof for the manufacture of a medicament for treating and/or preventing a demyelinating disorder of the CNS in said individual.

Similarly, the instant invention also pertains to the use of a compound suitable for generating an immune reaction directed against α1-3-galactose in an individual in need thereof for treating and/or preventing a demyelinating disorder of the CNS in said individual.

Within the scope of the invention, the expression "generating an immune reaction" encompasses an antigen-antibody reaction and an antibody-mediated activation of the immune system.

In other words, the expression "a compound suitable for generating an immune reaction directed against α1-3-galactose" intends to mean that the α1-3-galactose is the epitope of interest which is intended to be targeted by the immune system.

Within the scope of the instant invention, the term "treatment" refers to a therapy intended to cure, attenuate or alleviate at least one symptom observed in a demyelinating disorder of the CNS.

As used herein, the term "prevention" refers to the reduction of the risk of onset or slowing the occurrence of a demyelinating disorder of the CNS.

In other words, the term "preventing" also encompasses "reducing the likelihood of occurrence" or "reducing the likelihood of reoccurrence" of the demyelinating disorder of the CNS.

In some embodiments, the α1-3-galactose epitope comprises a galactose-α-1-3-galactose-β-1-4-N-acetylglucosamine moiety (Galα1-3Galβ1-4GlcNAc).

In some embodiments, the α1-3-galactose epitope consists in the galactose-α-1-3-galactose-β-1-4-N-acetylglucosamine moiety (Galα1-3Galβ1-4GlcNAc).

In some embodiments, the said compound is either (1) a carrier presenting the epitope to the immune system, i.e. an epitope-associated carrier, in particular intended to generate antibodies directed against the said epitope, or (2) antibodies or a fragment thereof that specifically binds to the said epitope and is capable of mediating an immune system.

Within the scope of the instant disclosure, the expression "epitope-associated carrier" is intended to refer to an epitope that is linked, through a covalent or a non-covalent liaison, to a suitable carrier.

In certain embodiments, the above-defined compound is selected in a group comprising a α1-3-galactose-associated carrier and an antibody directed against α1-3-galactose or a fragment thereof.

In certain other embodiments, the α1-3-galactose-associated carrier may comprise a protein, a lipid, a polysaccharide, a glycoprotein, a glycolipid, a lipoprotein, a virus, a cell, a fraction thereof or a mixture thereof.

Illustratively, the α1-3-galactose epitope may be at the surface of a virus intended to be of use as a vaccine, such as an Influenza virus vaccine, as disclosed by Abdel-Motal et al. (J. virol. (2007) 9131-9141); the HIV virus vaccine, as disclosed by Abdel-Motal et al. (J. virol. (2006) 6943-6951; Vaccine (2010) 28(7):1758-1765).

In some embodiments, the cell is a probiotic bacterium.

In some particular embodiments, the probiotic bacterium possesses an α1-3-galactosyltransferase activity.

The α1-3-galactosyltransferase activity may be measured by the mean of standard protocols available in the state of the art. Illustratively, the α1-3-galactosyltransferase activity may be measured by ELISA (Keshvara et al.; Glycoconj. J. (1992) 9(1):16-20)), or by the mean of the protocol disclosed in Blanken and Van den Eijnden (J. Biol. Chem. (1985) 260(24):12927-12934) or Larsen et al. (Proc. Natl. Acad. Sci. (1989) 86:8227-8231).

In the later embodiments, the probiotic bacterium is selected in a group comprising a bacterium of the genus *Bacteroides* and a bacterium of the genus *Faecalibacterium.*

In some embodiments, the bacterium of the genus *Bacteroides* is selected in a group comprising *Bacteroides acidifaciens, B. amylophilus, B. asaccharolyticus, B. barnesiae, B. bivius, B. buccae, B. buccalis, B. caccae, B. capillosus, B. cellulosilyticus, B. cellulosolvens, B. clarus, B. coagulans, B. coprocola, B. coprophilus, B. coprosuis, B. corporis, B. denticola, B. disiens, B. distasonis, B. dorei, B. eggerthii, B. endodontalis, B. faecichinchillae, B. faecis, B. finegoldii, B. fluxus, B. forsythus, B. fragilis, B. furcosus, B. galacturonicus, B. gallinarum, B. gingivalis, B. gracilis, B. graminisolvens, B. helcogenes, B. heparinolyticus, B. hypermegas, B. intermedius, B. intestinalis, B. levii, B. loescheii, B. macacae, B. massiliensis, B. melaninogenicus, B. melaninogenicus intermedius, B. melaninogenicus macacae, B. melaninogenicus melaninogenicus, B. microfusus, B. multiacidus, B. nodosus, B. nordii, B. ochraceus, B. oleiciplenus, B. oralis, B. oris, B. oulorum, B. ovatus, B. paurosaccharolyticus, B. pectinophilus, B. pentosaceus, B. plebeius, B. pneumosintes, B. polypragmatus, B. praeacutus, B. propionicifaciens, B. putredinis, B. pyogenes, B. rodentium, B. ruminicola, B. ruminicola brevis, B. ruminicola ruminicola, B. salanitronis, B. salivosus, B. salyersiae, B. sartorii, B. splanchnicus, B. stercorirosoris, B. stercoris, B. suis, B. tectus, B. termitidis, B. thetaiotaomicron, B. uniformis, B. ureolyticus, B. veroralis, B. vulgatus, B. xylanisolvens, B. xylanolyticus, B. zoogleoformans* and a mixture thereof.

In some preferred embodiments, the bacterium of the genus *Bacteroides* is selected in a group comprising *B. coprocola, B. coprophilus, B. stercoris, B. xylanisolvens* and a mixture thereof.

In some embodiments, bacterium of the genus *Faecalibacterium* is *F. prausnitzii.*

Within the scope of the instant disclosure, the term "antibody", as used herein, is intended to refer to a molecule having a useful antigen binding specificity.

In other words, the antibody directed against α1-3-galactose according to the invention may specifically bind to antigenic Galα1-3Ga1β1-4GlcNAc epitope.

In certain embodiments, the antibody directed against α1-3-galactose is selected in a group comprising an IgG, an IgM, an IgA, and an IgD.

In particular embodiments, the antibody directed against α1-3-galactose is an IgG.

As used herein, the expression "antibody fragment" refers to fragment of antibody that maintain its capacity to bind the epitope, and may include Fab, F(ab')2 and Fv fragments.

In certain embodiments, the term "antibody" also includes genetically engineered derivatives of antibodies such as single chain Fv molecules (scFv) and domain antibodies (dAbs).

In some other embodiments, the antibody directed against α1-3-galactose may further include monoclonal antibodies, polyclonal antibodies, chimeric antibodies, humanized antibodies, human antibodies obtained by recombinant technologies.

The antibodies encompassed within the scope of the instant invention may be obtained by any suitable technique known in the state of the art, taking into consideration the good practices and the requirements in the field.

### • Demyelinating disorder of the CNS

In certain embodiments, the demyelinating disorder of the CNS is selected in a group comprising a clinically isolated syndrome (CIS) and a multiple sclerosis (MS).

Pre-diagnosis of the demyelinating disorder of the CNS may be performed according to the standard procedures currently implemented.

For example, MRI, electrical tests and blood analysis may be performed to identify patients having a demyelinating disorder of the CNS.

### COMPOSITIONS

### • Pharmaceutical and vaccine compositions

An aspect of the invention further relates to a pharmaceutical composition comprising (i) a compound according to the instant disclosure and a (ii) pharmaceutical acceptable vehicle. As used herein, the term "pharmaceutical acceptable" refers to any compound, material, excipient, composition or dosage form which are, under the rules and requirements of the medical practice, suitable for being in contact with the tissues and/or the organs of human and animal individuals without excessive toxicity, irritation, allergic response or other problem complications commensurate with a reasonable benefit/risk ratio.

The formulations of pharmaceutical compositions suitable to implement the disclosed invention may be obtained by following the routine and commons methods and principles in the art.

In some embodiments, a suitable pharmaceutically acceptable vehicle according to the invention may include any conventional solvents, dispersion media, fillers, solid carriers, aqueous solutions, coatings, antibacterial and antifungal agents, isotonic and absorption delaying agents, and the like.

In certain embodiments, suitable pharmaceutically acceptable vehicles may include, water, saline, phosphate buffered saline, dextrose, glycerol, ethanol and a mixture thereof.

In some embodiments, pharmaceutically acceptable vehicles may further comprise minor amounts of auxiliary substances such as wetting or emulsifying agents, preservatives or buffers, which enhance the shelf life or effectiveness of the cells.

Except insofar as any conventional media or agent is incompatible with the active ingredient, use thereof in the pharmaceutical compositions of the present invention is contemplated.

The instant invention also relates to a vaccine composition comprising (i) a compound or a pharmaceutical composition in accordance to the disclosure herein and (ii) an adjuvant.

In some embodiments, the adjuvant may be selected in a non-limited group comprising an aluminium salt, in particular aluminium phosphate and aluminium hydroxide; a squalene, a derivative thereof and a mixture thereof, in particular SAF-1; a muramyl peptide; a saponin derivative; a mycobacterium cell wall preparation; a monophosphoryl lipid A; a mycolic acid and a derivative thereof; a non-ionic block copolymer surfactant; Quil A; a cholera toxin B subunit; a polphosphazene and a derivative thereof; and an immunostimulating complexe (ISCOM), in particular a complex as described by Takahashi et al. (Nature; 1990, 344:873-875).

### METHODS

The methods disclosed herein may be achieved *in vitro, in vivo* or *ex vivo.*

### • Methods for the diagnosis of demyelinating disorders of the CNS

Another aspect of the invention relates to a method for diagnosing a demyelinating disorder of the CNS in an individual comprising the steps of:
- a) measuring the level of antibodies directed against α1-3-galactose in a sample from an individual;
- b) comparing the level of antibodies directed against α1-3-galactose measured at step a) to a reference value of the said level of antibodies directed against α1-3-galactose. In some embodiments, the sample is selected in a group comprising a whole blood sample, a plasma sample, a serum sample, a urine sample, a saliva sample, faeces sample.

In certain embodiments, the individual is a human or non-human mammal, preferably a human.

Within the scope of the instant invention, a non-human mammal includes a mammal comprising, a pet such as a dog, a cat, a domesticated pig, a rabbit, a ferret, a hamster, a mouse, a rat and the like; a primate such as a chimp, a monkey, and the like; an economically important animal such as cattle, a pig, a rabbit, a horse, a sheep, a goat.

In certain embodiments, the method further comprises step c) of concluding of the occurrence of a demyelinating disorder of the CNS from the comparison performed at step b).

In certain embodiments, the measurement of the level of the antibodies directed against α1-3-galactose is performed by ELISA.

Within the scope of the invention, any antibodies, in particular immune-globulins (Igs), are encompassed by the instant disclosure, preferably circulating Igs.

In some embodiments, the antibodies directed against α1-3-galactose are selected in a group comprising IgGs, IgMs, IgAs an IgDs.

In some embodiments, the antibodies directed against α1-3-galactose are IgGs.

In certain embodiments, a reference level value of the level of antibodies directed against α1-3-galactose may be measured from one or more biological sample(s) obtained from one healthy individual or from one or more biological sample(s) obtained from a population of healthy individuals, namely individuals not suffering from a demyelinating disorder of the CNS.

In some embodiments, a reference level value of the level of antibodies directed against α1-3-galactose may be measured from one or more biological sample(s) obtained from one individual or from one or more biological sample(s) obtained from a population of individuals, which individual(s) are known to suffer from a demyelinating disorder of the CNS.

In certain other embodiments, a reference level value of the level of antibodies directed against α1-3-galactose may be measured from one or more biological sample(s) obtained from one individual or from one or more biological sample(s) obtained from a population of individuals, which individual(s) are known to suffer from a demyelinating disorder of the CNS and being administered with a treatment against said disorder.

In certain other embodiments, a reference level value of the level of antibodies directed against α1-3-galactose may be obtained by calculating a mean value resulting from a compilation of data gathered from a database.

### • Methods for the treatment of demyelinating disorders of the CNS

Another aspect of the invention relates to a method for treating a demyelinating disorder of the CNS in an individual in need thereof comprising the administration to the said individual of a compound suitable for generating an immune reaction directed against α1-3-galactose, as defined herein.

A still another aspect of the invention also relates to a method for treating a demyelinating disorder of the CNS in an individual in need thereof comprising the administration to the said individual of a compound selected in a group comprising a of α1-3-galactose-associated carrier and an antibody directed against α1-3-galactose or a fragment thereof.

Another aspect of the invention further relates to a method for increasing the levels of antibodies directed against α1-3-galactose in an individual in need thereof comprising the administration to the said individual of a compound selected in a group comprising a α1-3-galactose-associated carrier and an antibody directed against α1-3-galactose or a fragment thereof.

Within the scope of the instant invention, individual in need thereof, i.e. an individual in need of an increased level of antibodies directed against α1-3-galactose, may be defined as having an expression level of said antibodies that is lower than the average level found in the age/gender match controls, said average level being representative as a reference value.

In some embodiments, the level of antibodies directed against α1-3-galactose may be at least 100 fold, 75 fold, 50 fold, 25 fold, 10 fold, 9 fold, 8 fold, 7 fold, 6 fold, 5 fold, 4 fold, 3 fold, 2 fold, 1.5 fold, 1.4 fold, 1.3 fold, 1.2 fold lower than a reference value.

In some embodiments, the individual in need thereof may be diagnosed by the mean of a method as disclosed herein.

Illustratively, another aspect of the invention hence relates to a method for diagnosing and treating a demyelinating disorder of the CNS in an individual comprising the steps of:
- a) performing a diagnostic method comprising the steps of:
   - a1) measuring the level of antibodies directed against α1-3-galactose in a sample from an individual;
   - a2) comparing the level of antibodies directed against α1-3-galactose measured at step a) to a reference value of the said level of antibodies directed against α1-3-galactose;
- b) determining whether said individual has a demyelinating disorder of the CNS when the measured level of antibodies directed against α1-3-galactose is lower than said reference value; and
- c) administering to the said individual a compound selected in a group comprising a α1-3-galactose-associated carrier and an antibody directed against α1-3-galactose or a fragment thereof.

In some embodiments, the compound suitable for generating an immune reaction directed against α1-3-galactose, the pharmaceutical composition or the vaccine composition, as disclosed herein, may be administered to an individual in need thereof by any route, i.e. by an oral administration, a topical administration, rectal administration or a parenteral administration, e.g., by injection, including a sub-cutaneous administration, a venous administration, an arterial administration, in intra-muscular administration, an intra-ocular administration, an intra-intestinal administration and an intra-auricular administration.

Illustratively, when the compound suitable for generating an immune reaction directed against α1-3-galactose is a probiotic bacterium or a mixture of probiotic bacteria, said probiotic bacterium or said mixture of probiotic bacteria may be introduced in the individual's intestine by the means disclosed in the state of the art.

This includes an oral administration of the said probiotic bacterium or mixture of probiotic bacteria in the form of an ingestible support, which may be selected in a group comprising a tablet, a coated tablet, a capsule, a syrup and a food product.

This also includes an intra-intestinal administration of the said probiotic bacterium or mixture of probiotic bacteria by the mean of endoscopy, rectal administration of capsule or suppository, or direct injection of an adequate liquid or solid formulation in the intestine.

In certain embodiments, the administration of the compound suitable for generating an immune reaction directed against α1-3-galactose, the pharmaceutical composition or the vaccine composition, as disclosed herein, by injection may be directly performed in the target tissue of interest, e.g. the intestine, in particular in order to avoid spreading of the said product.

Other suitable modes of administration may also employ pulmonary formulations, suppositories, and transdermal applications.

In some embodiments, an oral formulation according to the invention includes usual excipients, such as, for example, pharmaceutical grades of mannitol, lactose, starch, magnesium stearate, sodium saccharine, cellulose, magnesium carbonate, and the like.

In some embodiments, an effective amount of said compound is administered to said individual in need thereof.

Within the scope of the instant invention, an "effective amount" refers to the amount of said compound that alone stimulates the desired outcome, i.e. alleviates or eradicates the symptoms of the encompassed demyelinating disorder.

It is within the routine and the common knowledge of a skilled artisan to determine the effective amount of the compound suitable for generating an immune reaction directed against α1-3-galactose, the pharmaceutical composition or the vaccine composition, as disclosed herein, in order to observe the desired outcome.

Within the scope of the instant invention, the effective amount of the product to be administered may be determined by a physician or an authorized person skilled in the art and can be suitably adapted within the time course of the treatment.

In certain embodiments, the effective amount to be administered may depend upon a variety of parameters, including the material selected for administration, whether the administration is in single or multiple doses, and the individual's parameters including age, physical conditions, size, weight, gender, and the severity of the disease to be treated.

In certain embodiments, an effective amount of the compound suitable for generating an immune reaction directed against α1-3-galactose, as the main active agent, the pharmaceutical composition or the vaccine composition as disclosed herein, may comprise from about 0.001 mg to about 3000 mg of the active agent, per dosage unit, preferably from about 0.05 mg to about 100 mg, per dosage unit.

Within the scope of the instant invention, from about 0.001 mg to about 3000 mg of the active agent includes, from about 0.002 mg, 0.003 mg, 0.004 mg, 0.005 mg, 0.006 mg, 0.007 mg, 0.008 mg, 0.009 mg, 0.01 mg, 0.02 mg, 0.03 mg, 0.04 mg, 0.05 mg, 0.06 mg, 0.07 mg, 0.08 mg, 0.09 mg, 0.1 mg, 0.2 mg, 0.3 mg, 0.4 mg, 0.5 mg, 0.6 mg, 0.7 mg, 0.8 mg, 0.9 mg, 1 mg, 2 mg, 3 mg, 4 mg, 5 mg, 6 mg, 7 mg, 8 mg, 9 mg, 10 mg, 20 mg, 30 mg, 40 mg, 50 mg, 60 mg, 70 mg, 80 mg, 90 mg, 100 mg, 150 mg, 200 mg, 250 mg, 300 mg, 350 mg, 400 mg, 450 mg, 500 mg, 550 mg, 600 mg, 650 mg, 700 mg, 750 mg, 800 mg, 850 mg, 900 mg, 950 mg, 1000 mg, 1100 mg, 1150 mg, 1200 mg, 1250 mg, 1300 mg, 1350 mg, 1400 mg, 1450 mg, 1500 mg, 1550 mg, 1600 mg, 1650 mg, 1700 mg, 1750 mg, 1800 mg, 1850 mg, 1900 mg, 1950 mg, 2000 mg, 2100 mg, 2150 mg, 2200 mg, 2250 mg, 2300 mg, 2350 mg, 2400 mg, 2450 mg, 2500 mg, 2550 mg, 2600 mg, 2650 mg, 2700 mg, 2750 mg, 2800 mg, 2850 mg, 2900 mg and 2950 mg of the active agent, per dosage unit.

In certain embodiments, the of the compound suitable for generating an immune reaction directed against α1-3-galactose, the pharmaceutical composition or the vaccine composition may be administered at dosage levels sufficient to deliver from about 0.001 mg/kg to about 100 mg/kg of the active agent, from about 0.01 mg/kg to about 50 mg/kg, preferably from about 0.1 mg/kg to about 40 mg/kg, preferably from about 0.5 mg/kg to about 30 mg/kg, from about 0.01 mg/kg to about 10 mg/kg, from about 0.1 mg/kg to about 10 mg/kg, and more preferably from about 1 mg/kg to about 25 mg/kg, of subject body weight per day.

In some embodiments, the said treatment may be co-administered with another treatment consisting in the administration of one or more compounds selected in a group comprising prednisone, methylprednisolone, ocrelizumab, beta interferons, glatiramer acetate, dimethyl fumarate, fingolimod, teriflunomide, natalizumab, alemtuzumab, mycophenolate mofetil, mitoxantrone, and a mixture thereof.

### • Methods for the assessment of the efficiency of a treatment of a demyelinating disorder of the CNS

Another aspect of the invention relates to a method for assessing the efficiency of a treatment of a demyelinating disorder of the CNS in an individual, comprising the steps of:
- a) measuring the level of antibodies directed against α1-3-galactose in a biological sample from an individual prior to a treatment;
- b) measuring the level of antibodies directed against α1-3-galactose in a biological sample from an individual after a treatment;
- c) comparing level value measured at step a) with the level value measured at step b).

In some embodiments, the sample is selected in a group comprising a whole blood sample, a plasma sample, a serum sample, a urine sample, a saliva sample, faeces sample.

In some embodiments, the said treatment includes an administration of one or more compounds selected in a group comprising prednisone, methylprednisolone, ocrelizumab, beta interferons, glatiramer acetate, dimethyl fumarate, fingolimod, teriflunomide, natalizumab, alemtuzumab, mycophenolate mofetil, mitoxantrone, and a mixture thereof.

In certain embodiments, the method further comprises step d) consisting of adapting the treatment.

In some embodiments, any variation of the level value measured after the treatment with respect to the level value measured prior to the treatment may indicate the efficiency of said treatment.

In some embodiments, said method may be of use for helping the physician adjusting the treatment dosage, namely increasing or decreasing the dosage regimen to be administered.

### EXAMPLES

### 1- Materials and methods

### 1.1- Patients with multiple sclerosis (MS) or clinically isolated syndrome (CIS)

### 1.1.1 - First cohort of MS patients

A first cohort of 55 patients with MS (including 9 from twins) diagnosed at the Nantes and Toulouse University Hospitals was included in the study (see Table 1 for an excerpt of patients included in the study).

| **ID** | **Gender⁽¹⁾** | **Age** | **Group⁽²⁾** | **Diagnosis⁽³⁾** |
|---|---|---|---|---|
| **102** | M | 71 | NINDC dementia | Lewv body disease |
| **154** | M | 58 | NINDC non dementia | ALS |
| **191** | F | 57 | NINDC non dementia | Epilepsy |
| **376** | F | 63 | NINDC non dementia | ALS |
| **276** | F | 38 | NINDC non dementia | ALS |
| **296** | M | 54 | NINDC non dementia | Cognitive decline linked to NTH |
| **298** | F | 68 | NINDC non dementia | ALS |
| **511** | F | 36 | NINDC non dementia | Intracranial hypertension |
| **1100** | F | 50 | NINDC | narcolepsy |
| **104** | M | 49 | NINDC | cognitive trouble and depression |
| **201** | M | 36 | SC | Sensitive deficit of right leg of undetermined etiology |
| **224** | F | 43 | SC | Sensory symptoms |
| **233** | F | 47 | SC | Sensory symptoms, probably of vascular origin |
| **236** | F | 44 | SC | Bell's palsy |
| **239** | F | 54 | SC | Sensory symptoms, probably of vascular origin |
| **245** | F | 43 | SC | Sensory deficit of undetermined etiology |
| **289** | F | 47 | SC | Paresthesis + hemicorporeal right weakness |
| **258** | M | 51 | SC | Bell's palsv |
| **361** | F | 44 | SC | Sensory disorders of undertermined etiology |
| **366** | F | 37 | SC | Sensory disorders of undertermined etiology |
| **384** | F | 32 | SC | Functional disorders |
| **257** | F | 86 | | Instability and vertigo |
| **281** | M | 81 | | Partial epilepsy |
| **241** | M | 53 | | Cognitive dysfunction in the context of depression |
| **243** | F | 58 | | Cognitive dysfunction in the context of anxiety |
| **209** | F | 23 | | Vascular (Stroke) |
| **218** | F | 61 | | Brain lymphoma |
| **222** | F | 27 | | Cognitive dysfunction, post-traumatic |
| **323** | F | 34 | | Idiopathic intracranial hypertension |
| **324** | F | 46 | | Partial epilepsy |
| **333** | M | 37 | | Cerebellar ataxia secondary to ethylism |
| **341** | F | 73 | | Cerebral lymphoma |
| **357** | M | 47 | | Idiopathic intracranial hypertension |
| **389** | M | 59 | | Vascular cognitive disorders (non-Alzheimer's) |

| | | | | |
|---|---|---|---|---|
| ⁽¹⁾ M stands for "male" and F stands for "female"; ⁽²⁾ NINDC stands for "Non inflammatory neurological disease controls"; SC stands for "Symptomatic controls"; ⁽³⁾ ALS stands for "Amyotrophic lateral sclerosis". | | | | |

For the 46 non-twin patients, the gender ratio was 30F/16M and the average age of 47.6 years (range: 22 to 73). Thirty patients presented a remitting relapsing (RR) form (63.04%) and 16 presented a secondary or primary progressive (SP/PP) form (34.78%) of the disease. Patients received either no therapy (n=19, 41.3%) or IFNs (n=11, 23.9%), Glatiramer Acetate (n=1, 2.17%), Natalizumab or Fingolimod (n=7, 15.21%), Mycophenolate Mofetil (n=7, 15.21%) or Prednisone (n=1, 2.17%). The 9 patients from the twin pairs, with one patient of the pair having MS, were diagnosed at the University Hospital of Toulouse, the average age was 49.7 years (from 22 to 69), and all were monozygotic females (Table 1).

### 1.1.2 - Second cohort of MS patients

A second cohort consisted of 21 pregnant patients with MS obtained from the Lyon University Hospital. The average age was of 31.4 years (range 25-37) (Table 1). A total of 42 blood samples, stored at the Neurobiotec bank (biological resource center of the Lyon University Hospital) was obtained from these women, with one sample obtained during pregnancy and another one post-partum. Seven patients presented a post-partum relapse (from 3 weeks to 7 months post-partum). Pregnant women had taken no drugs, and post-partum medications are given in Table 1. Sera from women with MS were compared to samples from a specific control group of healthy pregnant women, matched for age and also for the trimester of pregnancy or post-partum at harvesting (n=42, ratio 1/1). In addition, the samples of healthy pregnant women were compared to samples of healthy non pregnant women (n=21) obtained from the Pediatric Department of Nantes University Hospital and blood bank of Rennes, respectively. Altogether, the MS cohort was composed of 76 patients (age mean: 43.2 years, from 22 to 73 years).

### 1.1.3 - Cohort of CIS patients

The last cohort consisted of 73 patients presenting with a clinically isolated syndrome (CIS) from the Université Catholique de Louvain (UCL). CIS was defined as a first mono- or multifocal neurological deficit lasting for more than 24 h, not associated with fever or infection, and compatible with a first presentation of MS Miller et al.; Lancet Neurol. 4 (2005) 341-348; McDonald et al.; Ann. Neurol. 50 (2001) 121-127). The gender ratio was 56F/17M, and the average age was 42 ± 1.2 years (range: 22 to 78). None of the CIS patients were under treatment at the time of blood harvesting.

### 1.1.4 - Cohort of non-MS/CIS patients

Finally, anti-Gal levels were also tested in a last cohort of 34 patients with non -MS/CIS pathologies of the central nervous system (CNS) (gender ratio: 11M/23F; age average: 50, from 23 to 86 years) according to the classification of Teunissen et al. (Mult. Scler. J. 19 (2013) 1802-1809) and compared to age and sex-matched normal individuals (see Figure 4).

### 1.2 - Age and sex paired controls, sampling and ethics:

To decrease experimental variables as much as possible, anonymous test tubes were coded and dispatched by a third party who was unaware of the study to allow an appropriate division of controls and experimental samples for serum testing. All ELISAs were thus carried out blind. Each sample from MS, CIS, or non-MS/CIS central nervous system diseases was compared to a sample from a normal individual paired for age and gender. All patients and controls signed an informed consent for the experiments.

### 1.3 - Antibodies

### 1.3.1 - Anti-α1,3 Gal antibodies

The ELISA was adapted from Buonomano et al. (Xenotransplantation. 6 (1999) 173-180). Plates were coated with Gal1-3Gal-polyacrylamide conjugate (5 µg/ml, PAA-Bdi; Lectinity, Moscow, Russia) overnight at 4°C, and then blocked with PBS 0.5% fish gelatin (Sigma-Aldrich) for two hours at 37°C. Human sera (at 1:1000, 1:2000 and 1:4000 in PBST) were incubated for two hours at 37°C. According to the quantity of sera available, a rabbit anti-human IgG/M followed by a HRP-goat anti-rabbit antibody (1:2000 and 1:2000, respectively; Jackson ImmunoResearch) or a HRP-donkey anti-human IgG (1:2000; Jackson ImmunoResearch) were used as secondary antibodies. A titration curve used serial dilutions of human IVIg (Privigen) at a first concentration of 200 ng/ml. For anti-Gal IgM ELISA, extensive washing with PBS - 0.1% Tween20 was performed between each step, and we developed the assay using HRP-F(ab')2-anti-human IgM (1:1000; Jackson Immunoresearch). Serial dilutions of purified IgM at concentrations starting at 800 ng/ml served as standards.

### 1.3.2- Anti-Neu5Gc antibodies

Anti-Neu5Gc were used as control since they are quantitatively the second source of "natural" antibodies directed against antigens missing in humans following the moss mutation of the CMAH gene. However immunization against Neu5Gc is related to animal meat and milk diet, human microbiota do not contain the CMAH gene, a situation which is thus opposite compared to the Gal one.

The dosage used an ELISA using mouse serum proteins as coating antigens according to Padler-Karavani et al. (PLoS ONE. 8 (2013)). Briefly, the plates were coated overnight at 4°C with wild-type mouse serum at a concentration of 1 µg/well diluted in 50 µl of coating buffer (50 mM sodium carbonate-bicarbonate buffer, pH 9.0). Diluted human sera were pre-incubated for two hours at 4°C with CMAH-KO mouse serum diluted 1:4000 in PBSTO (PBS 1X - 0.05% Tween 20 - 1% Ovalbumin; Sigma Aldrich). After washing and blocking, pre-treated human sera were added to the plates for two hours at room temperature (RT). IgGs and IgMs were detected by HRP-goat anti-human IgGs or PO-rabbit anti-Human IgMs (Dako). Then, TMB (Sigma Aldrich) was added, and the reaction was stopped by H₂SO₄. Optical density was read at 450 nm (630 nm as reference) using an MRX spectrophotometer (Dynatech laboratories, El Paso, US). Serial dilutions of IVIg (Privigen) or purified human IgM (Sigma) at concentrations starting at 800 ng/ml or 400 ng/ml, respectively, served as standards.

### 1.4 - Statistical analysis

### 1.4.1 - Metrology

Each ELISA was tested for reproducibility in duplicates in a panel of 90 samples. To assess the accuracy of the measurements, an intra-class correlation coefficient (ICC) and tested its statistical significance (H0: ICC=0.5; H1: ICC>0.5) using an F-test were computed. Both anti-Neu5Gc IgG and anti-Gal IgG or IgG/M tests were highly accurate (ICC=0.69 p=0.014 and ICC=0.83 p<0.001, respectively). In contrast, the anti-Neu5Gc IgM test had low reproducibility and was further excluded from the analysis (ICC=0.32 p=0.93).

### 1.4.2 - Statistics

For each antibody, the statistical significance of the comparisons were assessed using non-parametric matched pairs-Wilcoxon tests to account for any possible effect of age or gender on the observed traits (MS/CIS are age-dependent traits and gender is a well-established dimorphism in MS). Neu5Gc sensitization may be associated with age as observed in the controls (r= 0.14, p = 0.048). Statistical analyses were performed using Graphpad, Stata 13 SE and R statistical software.

### 2 - Results

### 2.1 - Anti-Neu5Gc and anti-Gal antibodies in a normal population

Since MS has a strong bias for gender and age, the initial design predicted a possible similar bias in the anti-Neu5Gc IgG, without any published reference values in the literature and with a trend for an effect of age in normal individuals. The option of comparing patients with age/gender matched normal individuals was thus retained. However, although the analyses were performed using age/gender matching values, the various patient cohorts (including MS and CIS) tested did not show a correlation with age/gender. Only healthy individuals showed a trend for a decrease of antibodies with age (p= 0.048). Figure 1 shows the distribution values of the antibodies in the healthy individuals (n= 120). The age average was 40.65 (range from 20 to 80 years). Means +/-SEM of anti-Neu5Gc IgG were 2.7 ± 0.3 ng/µl (Figure 1A) and 44.8 ± 8.1 ng/µl for anti-Gal IgG (Figure 1B). All normal individuals were found to be positive for anti-Neu5Gc IgG, consistent with a previous report Couvrat-Desvergnes et al. (J. Clin. Invest. 125 (n.d.) 4655-4665), and only 8% were negative for anti-Gal IgG.

### 2.2 - Lower anti-α 1,3 Gal Gal, with unmodified anti-Neu5Gc in patients with MS

Since IMN has been documented as a risk factor for MS and characterized by an overproduction of anti-Neu5Gc, anti-Neu5Gc were also measured in patients with MS and several MS patient sub-groups : a small cohort of twin patients who did or did not present with the disease (n=9), a group of RR (n= 30), of SP/PP form of MS (n=16), and a group of women (n= 21) during gestation and in the post-partum period who did (n=7) or did not present with a disease relapse in the months following delivery. No significant difference between the levels of anti-Neu5Gc of the MS patients compared to matched healthy controls was noted when all the MS patients (Figure 2A: 2.9 ± 0.5 ng/µl vs 2.3 ± 0.2 ng/µl; p=0.71) or the sub-groups was observed (data not shown).

However, we observed a significant drop in the mean anti-Gal IgG levels (29.2%) in the MS patients compared to their matched controls (Figure 2B: 9.2 ± 1.2 ng/µl vs 13 ± 1.6 ng/µl for controls; p= 0.03).

No significant results were found when the MS population was divided into the different subgroups, including comparisons between MS patients undergoing treatment or without treatment (Figure 3).

This significant decrease in anti-Gal was not observed in patients with non-MS central nervous system pathologies (Figure 4).

### 2.3 - Lower anti-Gal IgG with unmodified anti-Neu5Gc in clinically isolated syndrome

Anti-Neu5Gc and anti-Gal levels were also tested in a cohort of 73 patients with CIS tested independently after the MS cohort. As for overt MS disease, we found no difference in the anti-Neu5Gc IgG levels between CIS and the matched normal individuals (Figure 5A: p = 0.87). However, we also observed a significant decrease in the levels of the anti-Gal IgG (Figure 5B: 44.8 ± 7.1 ng/µl vs 64.7 ± 13.4 ng/µl; a 30.7 % decrease; p=0.02). Taken together, our data on CIS provides additional support for the new observation that patients with MS have a decrease in their anti-Gal IgG levels (combination of the two cohorts vs. matched controls: p=0.0027).

## Claims

1. A compound suitable for generating an immune reaction directed against α1-3-galactose in an individual in need thereof for use in treating and/or preventing a demyelinating disorder of the central nervous system (CNS) in said individual.

2. The compound for use according to claim 1, which is selected in a group comprising a α1-3-galactose-associated carrier and an antibody directed against α1-3-galactose or a fragment thereof.

3. The compound for use according to any one of claims 1 and 2, wherein the α1-3-galactose-associated carrier comprises a protein, a lipid, a polysaccharide, a glycoprotein, a glycolipid, a lipoprotein, a virus, a cell, a fraction thereof or a mixture thereof.

4. The compound for use according to claim 3, wherein the cell is a probiotic bacterium.

5. The compound for use according to claim 4, wherein the probiotic bacterium possesses an α1-3-galactosyltransferase activity.

6. The compound for use according to claim 5, wherein the said probiotic bacterium is selected in a group comprising a bacterium of the genus *Bacteroides* and a bacterium of the genus *Faecalibacterium.*

7. The compound for use according to any one of claims 1 and 2, wherein the antibody directed against α1-3-galactose is selected in a group comprising an IgG, an IgM, an IgA, and an IgD.

8. The compound for use according to any one of claims 1 to 7, wherein the demyelinating disorder of the CNS is selected in a group comprising a clinically isolated syndrome (CIS) and a multiple sclerosis (MS).

9. A pharmaceutical composition comprising a compound according to any one of claims 1 to 8 and a pharmaceutical acceptable vehicle.

10. A vaccine composition comprising a compound according to any one of claims 1 to 8 or a pharmaceutical composition according to claim 9 and an adjuvant.

11. A method for diagnosing a demyelinating disorder of the CNS in an individual comprising the steps of:
- a) measuring the level of antibodies directed against α1-3-galactose in a sample from an individual;
- b) comparing the level of antibodies directed against α1-3-galactose measured at step a) to a reference value of the said level of antibodies directed against α1-3-galactose.

12. The method according to claim 11, further comprising step c) of concluding of the occurrence of a demyelinating disorder of the CNS from the comparison performed at step b).

13. The method according to any one of claims 11 and 12, wherein the measurement of the level of the antibodies directed against α1-3-galactose is performed by ELISA.

14. The method according to any one of claims 11 to 13, wherein the antibodies directed against α1-3-galactose are selected in a group comprising IgGs, IgMs, IgAs an IgDs.

15. A method for assessing the efficiency of a treatment of a demyelinating disorder of the CNS in an individual, comprising the steps of:
- a) measuring the level of antibodies directed against α1-3-galactose in a biological sample from an individual prior to a treatment;
- b) measuring the level of antibodies directed against α1-3-galactose in a biological sample from an individual after a treatment;
- c) comparing level value measured at step a) with the level value measured at step b).
